(19) Europäisches Patentamt
European Patent Office
Office européen des brevets

(11) EP 3 265 461 B1

(12) EUROPEAN PATENT SPECIFICATION

(45) Date of publication and mention of the grant of the patent:
**23.10.2019 Bulletin 2019/43**

(21) Application number: **16712512.9**

(22) Date of filing: **25.02.2016**

(51) Int Cl.:
*C07D 407/12* (2006.01) *A61K 31/351* (2006.01)
*A61P 9/00* (2006.01)

(86) International application number:
**PCT/IB2016/051022**

(87) International publication number:
**WO 2016/142797 (15.09.2016 Gazette 2016/37)**

# (54) VERBASCOSIDE DERIVATIVES

VERBASCOSID-DERIVATE

DÉRIVÉS DE VERBASCOSIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.03.2015 IT MI20150334**

(43) Date of publication of application:
**10.01.2018 Bulletin 2018/02**

(73) Proprietor: **Croda Italiana S.p.A.**
**27036 Mortara (PV) (IT)**

(72) Inventors:
- **SGARAVATTI, Elena**
  **35123 Padova (PD) (IT)**
- **DAL TOSO, Roberto**
  **36051 Creazzo (VI) (IT)**
- **COPETTI, Stefano**
  **35136 Padova (PD) (IT)**

(74) Representative: **de Saint Viance, Isabelle Marie Fanny**
**Sederma**
**29, rue du chemin vert**
**78610 Le-Perray-en-Yvelines (FR)**

(56) References cited:
- **KHAZIR J. ET AL.: "Enzyme mediated-transesterification of verbascoside and evaluation of antifungal activity of synthesised compounds", NATURAL PRODUCT RESEARCH, vol. 29, no. 8, December 2014 (2014-12), pages 727-734, XP002741791,**
- **GIANLUCA CAMPO ET AL: "The in vitro effects of verbascoside on human platelet aggregation", JOURNAL OF THROMBOSIS AND THROMBOLYSIS, KLUWER ACADEMIC PUBLISHERS, BO, vol. 34, no. 3, 22 June 2012 (2012-06-22), pages 318-325, XP035117721, ISSN: 1573-742X, DOI: 10.1007/S11239-012-0757-Z**
- **PENNACCHIO MARCELLO ET AL: "Cardioactive compounds from Eremophila species", JOURNAL OF ETHNOPHARMACOLOGY, ELSEVIER IRELAND LTD, IE, vol. 53, no. 1, 1996, pages 21-27, XP009140452, ISSN: 0378-8741**
- **YUH-HWA LIU ET AL.: "Inhibitory activities of acteoside, isoacteoside, and its structural constituents against protein glycation in vitro", BOTANICAL STUDIES, vol. 54, no. 6, 2013, pages 1-9, XP002741792, DOI: 10.1186/1999-3110-54-6**
- **CAPASSO A ET AL: "The Effect of Phenylpropanoid Glycosides on Rabbit Platelet Aggregation In Vitro", PHARMACOLOGICAL RESEARCH, ACADEMIC PRESS, LONDON, GB, vol. 27, 1993, pages 113-114, XP024877540, ISSN: 1043-6618, DOI: 10.1006/PHRS.1993.1091 [retrieved on 1993-05-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 265 461 B1

## Description

### TECHNICAL FIELD

Derivatives of verbascoside and the pharmaceutical compositions containing them are the subject matter of the present invention.

### BACKGROUND ART

Cardiovascular diseases are a chronic pathology which develops insidiously during an individual's life and often it is symptomatic only when it has already reached an advanced stage. This pathology is still the leading cause of premature death in Western countries and in any case, due to its extensive dissemination, it often leads to mass disability because of its characteristic incapacitating symptomatology.

Primary prevention of cardiovascular diseases is one of the greatest challenges faced by the healthcare system. The main risk factors have been identified and guidelines to be followed by the population for a correct self-prevention have been formulated, however the fact is that, although a change in life style (movement, proper diet, etc.) is essential, very often it is not sufficient, thus needing a pharmacological treatment.

Unfortunately, the use of conventional drugs and therapies is limited in a primary prevention due to their side effects and costs. For example, statins are the most effective hypolipidemic drugs for the primary prevention of cardiovascular diseases and are generally well tolerated. Nevertheless, some patients show side effects such as high hepatic enzyme levels, gastrointestinal symptoms and myalgia, myositis and statin-induced rhabdomyolysis. In these patients, alternative therapies with natural substances have been proposed to keep hypercholesterolemia under control.

Similarly, the benefits of using aspirin or other platelet antiaggregants in the reduction of vascular events are to be carefully evaluated in light of the increased risk of bleeding and other side effects such as epistaxis, haematuria and easy bruising. In light of this evidence, aspirin and the other platelet antiaggregants are not recommended for the primary prevention of cardiovascular diseases.

Furthermore, aspirin is not effective in the treatment of diabetic patients due to its mechanism of action.

Verbascoside is a polyphenol of formula:

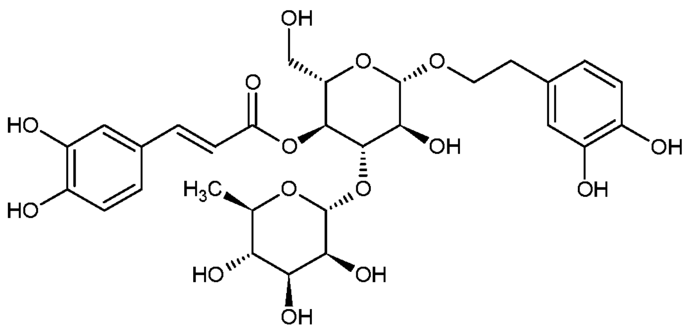

which may be extracted from plants or cell lines, such as *Syringa vulgaris,* and which has anti-oxidizing, cicatrizing, and cardio-protective activity.

### SUMMARY OF THE INVENTION

It has been found that verbascoside also has platelet antiaggregant activity via the modulation of two mechanisms, i.e. one involving arachidonic acid, cyclooxygenase and thromboxane A2, and a second one acting through ADP and P2Y12 receptor.

By virtue of this discovery, due to the double-action mechanism, it was seen that verbascoside can also be used as a platelet antiaggregant in the treatment of diabetic patients, who vice versa would not be responsive to a therapy with acetylsalicylic acid.

It has also been seen that said pharmacological activity is present in verbascoside derivatives of formula (I)

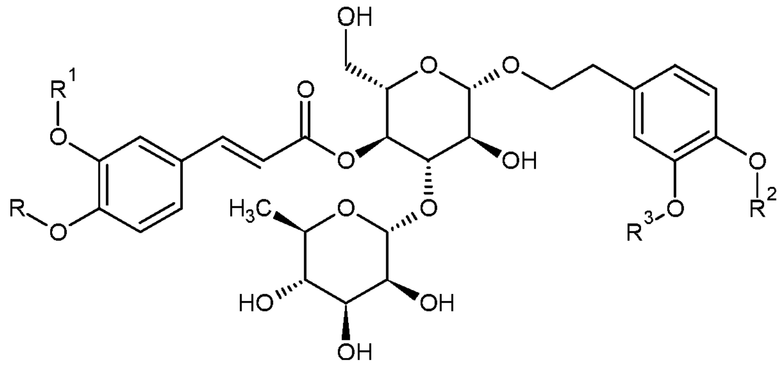

(I)

where R, $R^1$, $R^2$ and $R^3$ are equal or different and are independently selected from hydrogen and carboxylic acid residue, so that the ester groups R-O-, $R^1$-O-, $R^2$-O- and $R^3$-O-, respectively, can be hydrolyzed at a pH from 4.5 to 7, provided that R, $R^1$, $R^2$ and $R^3$ are not simultaneously hydrogen; and wherein at least one of R, $R^1$, $R^2$ and $R^3$ is a residue of pivalic acid.

It was seen that said derivatives may give rise to the formation of verbascoside *in vivo,* e.g. via the action of hepatic enzymes or, partially, in blood, with delayed-release kinetics. Nevertheless, with respect to verbascoside, said compounds have greater oxidation stability, such as that occurring in a normal aqueous saline solution, which allows a pharmaceutical formulation to be prepared in the form of a solution which remains stable over time.

This evidence allows the compounds of formula (I) to be designed as ideal verbascoside prodrugs.

Therefore, a compound of formula (I) as defined above, for use as a platelet antiaggregant, is a first object of the present invention.

The compounds of formula (I) as defined above, but with the condition that R, $R^1$, $R^2$, and $R^3$ are not simultaneously hydrogen, are a second object of the present invention.

The compounds of formula (I) as defined above, for use as platelet antiaggregants in the treatment of diabetic patients, in particular of diabetic patients at risk for cardiovascular diseases, are an additional object of the invention.

## DETAILED DESCRIPTION OF THE INVENTION

This invention relates to compounds of formula (I):

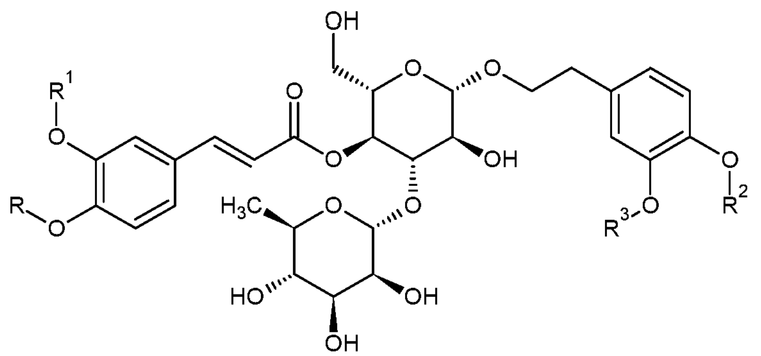

(I)

where R, $R^1$, $R^2$ and $R^3$ are equal or different and are independently selected from hydrogen and a carboxylic acid residue, so that the ester groups R-O-, $R^1$-O-, $R^2$-O- and $R^3$-O-, respectively, can be hydrolyzed at a pH from 4.5 to 7, provided that R, $R^1$, $R^2$ and $R^3$ are not simultaneously hydrogen; and wherein at least one of R, $R^1$, $R^2$ and $R^3$ is a residue of pivalic acid. for use as a platelet antiaggregant.

In certain embodiments, the ester groups R-O-, $R^1$-O-, $R^2$-O- and $R^3$-O-, respectively, are hydrolyzed at a pH from 5 to 6.5.

In other embodiments, the ester groups R-O-, $R^1$-O-, $R^2$-O- and $R^3$-O-, respectively, are hydrolyzed at a pH from 6 to 7, preferably from 6.3 to 6.7.

In certain embodiments, at least one of R, $R^1$, $R^2$ and $R^3$ is a pivalic acid residue.

In particularly preferred embodiments, at least R and $R^1$ or $R^2$ and $R^3$ are simultaneously a pivalic acid residue.

In another particularly preferred embodiment, R, $R^1$, $R^2$ and $R^3$ are simultaneously hydrogen.

The derivatives of formula (I) as defined above are a further object of the present invention, but with the condition that R, $R^1$, $R^2$ and $R^3$ are not simultaneously hydrogen.

The compounds of formula (I) may be prepared as shown below in the experimental part. In general, verbascoside may be reacted with an activated form of the selected carboxylic acid, such as a chloride or bromide of the acid, an anhydride or in the presence of condensing agents, according to the techniques typically used by an organic chemist.

The choice of the carboxylic acid which can be hydrolyzed in the above pH range can easily be made by means of a stability test at various pHs on the synthesized compound of formula (I), according to the traditional trial-and-error method.

Verbascoside is commercially available and may be obtained by means of extraction from plants or plant cell cultures which contain it. By way of example, verbascoside may be obtained from a selected cell culture of *Syringa vulgaris,* such as the cell line DSM 16857 deposited at DSMZ (Deutsche Sammlung Von Mikroorganismen und Zellkulturen, Braunschweig, Germany).

The compounds of formula (I) have shown a platelet antiaggregant activity which, when the groups R, $R^1$, $R^2$ and $R^3$ are not simultaneously hydrogen, according to an unconfirmed hypothesis, involves the *in vivo* release of verbascoside with delayed-release kinetics.

The compounds of formula (I) where the groups R, $R^1$, $R^2$ and $R^3$ are not simultaneously hydrogen have also shown a greater stability of verbascoside towards oxidation. Since an aqueous medium, such as a normal saline solution for example, is already capable of promoting the oxidation of verbascoside, such ester derivatives on the catecholic oxygens may be validly used in the place of verbascoside, especially when preparing pharmaceutical formulations in aqueous solution and in any case in normal saline medium, so as to allow the active ingredient to be released at the intestinal mucosa.

Moreover, the sustained release kinetics of the compounds where the R, $R^1$, $R^2$ and $R^3$ groups are not simultaneously hydrogen allows a once-a-day administration of the drug, with an obvious advantage for the patient.

The preferred compounds of formula (I) are those where R and $R^1$ or $R^2$ and $R^3$ are simultaneously a carboxylic acid residue which may be hydrolyzed at a pH from 4.5 to 7. Especially preferred are the compounds of formula (I) where R and $R^1$ or $R^2$ and $R^3$ are simultaneously a pivaloyl group.

Pharmaceutical compositions containing the compounds of formula (I) are a further object of the present invention.

The compositions of the invention can be prepared according to methodologies known to a person skilled in the art, such as those indicated in Remington's Pharmaceutical Sciences Handbook, Mack Pub. Co., N.Y., USA, 17th edition, 1985.

The compounds of formula (I) are contained in the compositions in weight percentages which may vary from 0.01%to 30%, preferably from 0.1% to 20%, more preferably from 1 %to 10%.

## PREPARATORY PART

Synthesis of bis-pivaloyl derivative of formula (Ia)

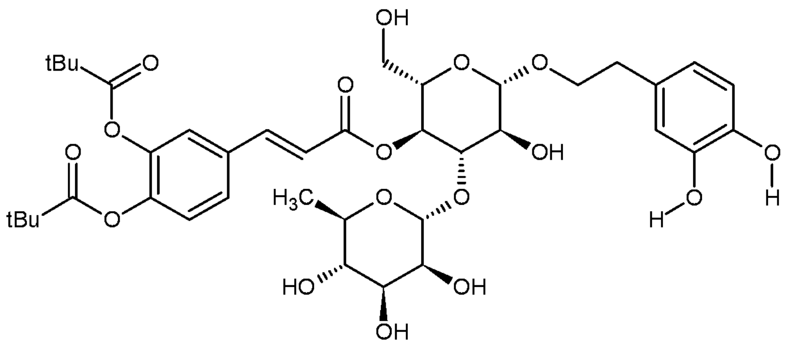

(Ia)

600 mg (0.96 mmol, 70% purity) of verbascoside are dissolved in anhydrous DMF (6 mL) under nitrogen atmosphere. Diisopropylethylamine (DIPEA, 1 mL, 6 eq, 5.76 mmol) and immediately afterwards 4-dimethylaminopyridine (DMAP, 235 mg, 2 eq, 1.92 mmol) are added. The yellow solution is cooled with an ice bath and then pivaloyl chloride

(208 mg, 1.8 eq, 1.73 mmol) dissolved in 0.5 mL of DMF is added. The reaction is left under stirring for 30 min. at 0°C, then it is poured into a saturated aqueous solution of ammonium chloride. The mixture is then extracted twice with ethyl ether/ethyl acetate 1/1 and the organic phase is dried with sodium sulfate, filtered and evaporated thus obtaining 520 mg of raw compound (Ia). The product is purified by means of preparative HPLC obtaining 150 mg of compound (Ia).
$^1$H-NMR (500 MHz, $^d$acetone) δ ppm 1.10 (d, J=5.87 Hz, 3H), 1.34 (s, 9H), 1.35 (s, 9H), 2.74-2.78 (m, 2H), 3.32 (t, J=9.20 Hz, 1H), 3.44 (t, J=8.20 Hz, 1H), 3.49-3.78 (m, 10H), 3.85-3.92 (m, 2H), 3.96-4.03 (m, 1H), 4.43 (d, J=8.20 Hz, 1H), 4.52 (br s, 1H), 4.93 (t, J=9.54 Hz, 1H), 5.30 (s, 1H), 6.54-6.60 (m, 2H), 6.73 (d, J=7.83 Hz, 1H), 6.76 (d, J=1.47 Hz, 1H), 7.29 (d, J=7.83 Hz, 1H), 7.59-7.65 (m, 2H), 7.65-7.84 (m, 3H).

Synthesis of tetra-pivaloyl derivative of formula (Ib)

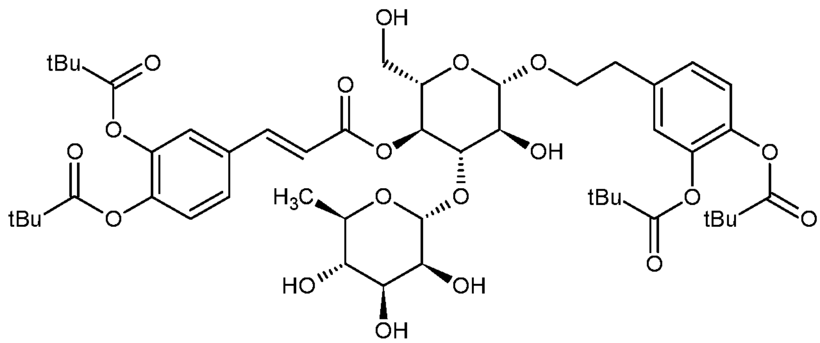

(Ib)

Verbascoside (600 mg, 0.96 mmol) was dissolved in anhydrous DMF (6 mL) under hydrogen atmosphere. DIPEA (12 eq, 11.52 mmol, 2 mL) was added, followed by DMAP (4 eq, 500 mg).

The yellow solution was cooled with an ice bath and treated with pivaloyl chloride (4 eq, 460 mg) dissolved in 0.5 mL of DMF.

The reaction was left under stirring at 0°C for 30 min, then it was poured into a saturated aqueous solution of $NH_4Cl$. The reaction mixture was then extracted twice with $Et_2O$/EtOAc 1/1 and the organic phase was dried over sodium sulfate, filtered and evaporated thus obtaining 750 mg of raw compound (Ib). The raw fraction was purified by means of preparative HPLC obtaining 240 mg of pure product.
$^1$H-NMR (500 MHz, $^d$acetone) δ 1.10 (d, J=6.40 Hz, 3H), 1.33 (s, 9H), 1.34 (s, 9H), 1.35 (s, 9H), 2.89-2.99 (m, 2H), 3.31 (d, J=9.20 Hz, 1H), 3.45 (t, J=8.20 Hz, 1H), 3.49-3.83 (m, 10H), 3.85-3.93 (m, 2H), 4.05-4.13 (m, 1H), 4.46 (d, J=8.20 Hz, 1H), 4.60 (br s,1H), 4.93 (t, J=9.78 Hz, 1H), 5.30 (s, 1H), 6.57 (d, J=16.14 Hz, 1H), 7.10 (d, J=8.31 Hz, 1H), 7.15 (d, J=1.96 Hz, 1H), 7.21 (dd, J=8.31-1.96 Hz, 2H), 7.29 (d, J=7.83 Hz, 1H), 7.59-7.65 (m, 2H), 7.71 (d, J=16.14 Hz, 1H).

## EXPERIMENTAL PART

A randomized, double-blind Phase II study was conducted to evaluate verbascoside activity in modulating the platelet aggregation (PA) values on subjects with at least one cardiovascular risk factor, selected from: age over 65 years, diabetes mellitus, hypertension, smoke, hyperlipidemia, waist circumference greater than 102 cm in men or 88 cm in women. Patients under pharmacological treatment with platelet antiaggregants (aspirin, clopidogrel, ticlopidine, prasugrel, ticagrelor), with anticoagulants (e.g. warfarin) or with oral contraceptives, were excluded. Other exclusion criteria were: events of myocardial infarction, coronary revascularization (percutaneous or surgical), infarction, temporary ischemic attack, peripheral vascular pathology, and the consumption of corticosteroids and/or non-steroidal anti-inflammatory drugs in the last month.

The experimentation was conducted on 100 subjects. The subjects were divided into three groups: one underwent a treatment with 50 mg/day of verbascoside (1 25-mg capsule twice a day); a second group underwent a treatment with 100 mg/day of verbascoside (2 25-mg capsules twice a day); the third group was treated with placebo (2 capsules twice a day). The treatment lasted 2 weeks. The subjects were randomly assigned to groups of four and divided into four categories: age over 60 years vs. age equal to or less than 60 years; male vs. female; current use of cigarettes yes vs. no; diabetes mellitus yes vs. no. These categories were generated because age, sex, smoking, and diabetes mellitus significantly influence PA.

The blood samples were drawn from an antecubital vein on day 0 (before taking the drug) and at the end of

the trial (week 2). The first 2-4 mL of blood were discarded to avoid the spontaneous activation of the platelets. All the subjects were sampled after at least 30 minutes of rest and 2 hours of fasting. Two 4.5 mL test tubes containing a 3.8% solution of sodium citrate were collected for each patient.

PA was determined by means of light transmission aggregometry according to standard protocols. The blood was centrifuged (200 revs/10 min.) obtaining a platelet-rich-plasma (PRP). The remaining sample was re-centrifuged (1550 revs/15 min.) obtaining a platelet-poor-plasma (PPP). The platelets were stimulated with 1 μM of arachidonic acid (AA) and 5 μM of adenosine diphosphate (ADP). The aggregation was measured at 37°C with a PACKS-4 aggregometer (Helena Laboratories). The curves were recorded for 6 min. The aggregation was measured at a maximum aggregation (maximum PA) and at 5 min (delayed PA). The inhibition of platelet aggregation (IPA) was defined as the percent decrease of the aggregation values (maximum PA and delayed PA) obtained at time 0 and after treatment: IPA(%) = $(PA_{time\ 0} - PA_{week\ 2} / PA_{time\ 0}$. The percent of platelet disaggregation (PD) between the values of maximum PA and delayed PA was defined as: PD (%) = 100 x $(1- PA_{delayed}/PA_{maximum})$. In order to define whether a significant variability was present among the various subjects in response to the antiplatelet treatment, a variation coefficient CV = SD/average was used. A significant variability was determined with CV >0.25.

Verbascoside was obtained from a selected cell culture of *Syringa vulgaris* (cell line DSM 16857 deposited at DSMZ (Deutsche Sammlung Von Mikroorganismen und Zellkulturen, Braunschweig, Germany).

## RESULTS

The following data were collected:

- values of maximum PA after 2 weeks of treatment
- delayed PA
- %IPA
- %PD.

Routine laboratory exams (hematology, clinical chemistry, etc.) and measurements of vital functions were also conducted on the subjects at both time 0 and at the end of the treatment to evaluate the onset of side effects.

The baseline PA values (PA at time 0) were assumed to be 50-60% with a standard deviation of 10-12%. A statistical analysis was conducted on the trial with STATISTICA 8 software (Statsoft Inc., Tulsa, Okla, USA).

### Arachidonic acid-induced maximum PA

No significant differences were revealed between the various subject populations (placebo 50±12% vs. 50 mg of verbascoside 58±8% vs. 100 mg of verbascoside 60±12%, p=0.5) at time 0. After 2 weeks of treatment with placebo, no significant change of the PA values was observed in comparison with time 0 (50±11% vs. 50±12%, p=0.8). The treatment with 50 mg/day of verbascoside did not produce substantial changes either (48±10% vs. 50±9%, p=0.4). Instead, the treatment with 100 mg/day of verbascoside for 2 weeks produced a substantial decrease in PA values (39±15% vs. 51±13%, p<0.01).

### ADP-induced maximum PA

No significant differences were observed between the various subject populations (placebo 58±12% vs. 50 mg of verbascoside 58±8% vs. 100 mg of verbascoside 60±12%, p=0.5) at time 0. After 2 weeks of treatment with placebo, no significant change of the PA values was observed in comparison with time 0 (58±13% vs. 58±12%, p=0.8). The treatment with 50 mg/day of verbascoside did not produce substantial changes either (56±9% vs. 58±8%, p=0.5). Instead, the treatment with 100 mg/day of verbascoside for 2 weeks produced a substantial decrease in PA values (49±15% vs. 60±12%, p<0.01).

### Oxidation stability of the compounds of formula (I)

The oxidation stability of the compounds of formula (I) was evaluated as a function of their antioxidant power, determined as the reactivity of the compound against the stable radical 2,2-diphenyl-1-picrylhydrazyl (DPPH) in solution. High reactivity means poor stability under oxidizing conditions.

A 100 μM solution of DPPH in methanol, prepared from a 1 mM solution, then diluted at 1:10 was used for the determination.

The following final dilutions (from a solution in methanol having a double concentration) of the compound of formula (I) to be analyzed were prepared: 500 μg/mL, 100 μg/mL, 50 μg/mL, 10 μg/mL, 5 μg/mL, 2.5 μg/mL, 1 μg/mL,

0.5 μg/mL, 0.1 μg/mL, 0.05 μg/mL, 0 μg/mL.

500 μl of the compound to be tested at a certain concentration and 500 μl of DPPH were placed in a cuvette, they were all mixed and incubated in the dark at room temperature for 15 min.

The solution was analyzed with a spectrophotometer at a wavelength of 515 nm (methanol as white).

The color in the cuvettes varies from violet (DPPH without compound of formula (I)) to straw-yellow (DPPH with maximum inhibitory concentration of the compound of formula (I)).

The percent inhibition of the radical reaction is defined as:

$$[(\text{Abs max (DPPH alone)} - \text{Abs (DPPH + compound)})/\text{Abs max}]\text{x}100.$$

The antiradical activity is defined as the concentration of antioxidant substance which lowers the initial concentration of DPPH by 50%.

The results are shown in Table I below.

Table I

| Compound | Inhibitory activity (IC50) in μg/mL |
|---|---|
| (A) Verbascoside ($R,R^1,R^2,R^3$=H) | 2 |
| (B) $R,R^1$=pivaloyl; $R^2,R^3$=H | 4.24 |
| (C) $R,R^1$=H; $R^2,R^3$=pivaloyl | 3.7 |
| (D) $R,R^1,R^2,R^3$=pivaloyl | Inactive |

The data show that the compounds of formula (I) where R, $R^1$, $R^2$, and $R^3$ are not all hydrogen have an oxidation stability much higher than verbascoside or are completely inactive.

Stability of the compounds of formula (I) under physiological conditions

The stability of the compounds of formula (I) was measured under physiological conditions.

The stability measurement was conducted by means of analytic HPLC techniques with a UV detector at 330 nm. The half-life is reported (in hours).

The data are shown in table II.

Table II

| Compound | SGF t1/2 (hours) | FASSIF t1/2 (hours) | FESSIF t1/2 (hours) | Verbascoside formulation | Blood t1/2 (hours) |
|---|---|---|---|---|---|
| (A) | STABLE | stable | >24 | - | stable |
| (B) | STABLE | 8 | 6 | yes | stable |
| (C) | STABLE | 7 | 5 | yes | stable |
| (D) | STABLE | 24 | stable | yes | 0.5 |
| SGF=stability in gastric fluids (pH=1.6)<br>FASSIF=stability in fasted intestinal fluid (pH=6.5)<br>FESSIF=stability in fed intestinal fluid (pH=5). | | | | | |

Moreover, it has also been determined that the derivatives (B), (C) and (D) are metabolized in human liver, whereas verbascoside (derivative (A)) is stable.

## Claims

**1.** Compounds of formula (I):

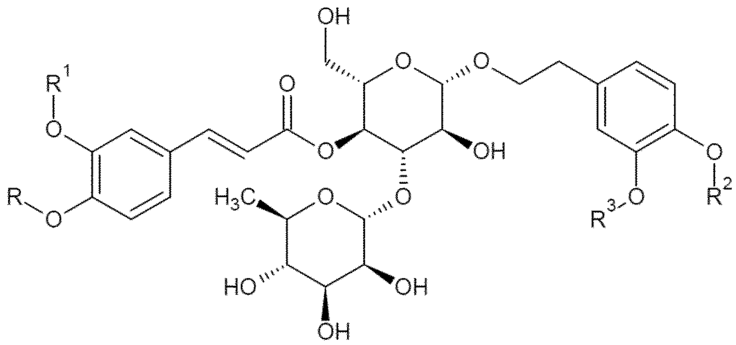

(I)

wherein R, $R^1$, $R^2$ and $R^3$ are the same or different and are independently selected from hydrogen and a residue of a carboxylic acid, wherein the ester groups R-O-, $R^1$-O-, $R^2$-O- and $R^3$-O-, respectively, are hydrolyzable to a pH ranging between 4.5 and 7, provided that R, $R^1$, $R^2$ and $R^3$ are not simultaneously hydrogen; and
wherein at least one of R, $R^1$, $R^2$ and $R^3$ is a residue of pivalic acid.

**2.** The compounds of formula (I) according to claim 1, wherein the ester groups R-O-, $R^1$-O-, $R^2$-O- and $R^3$-O-, respectively, are hydrolyzable to a pH ranging between 5 and 6.5, or to a pH ranging between 6 and 7, or to a pH ranging between 6.3 and 6.7.

**3.** The compounds of formula (I) according to claim 1 or 2, wherein at least R and $R^1$ or $R^2$ and $R^3$ are simultaneously a residue of pivalic acid.

**4.** The compounds of formula (I) according to claim 3, wherein R and $R^1$ are a residue of pivalic acid and $R^2$ and $R^3$ are H, or R and $R^1$ are H and $R^2$ and $R^3$ are a residue of pivalic acid.

**5.** A pharmaceutical formulation containing at least one compound of formula (I) according to any of the claims 1 to 4, and pharmaceutically acceptable excipients.

**6.** The compounds according to anyone of claims 1 to 4, or a composition according to claim 5, for use as platelet antiaggregants.

**7.** The compounds of formula (I) for use as defined in claim 6, wherein the ester groups R-O-, $R^1$-O-, $R^2$-O- and $R^3$-O-, respectively, are hydrolyzable to a pH ranging between 5 and 6.5, or to a pH ranging between 6 and 7, or to a pH ranging between 6.3 and 6.7.

**8.** The compounds of formula (I) for use as defined in claim 6 or 7, in the treatment of diabetic patients.

**9.** The compounds of formula (I) for use as defined in claim 8, in the treatment of diabetic patients at risk for cardiovascular diseases.

**10.** The compounds of formula (I) for use as defined in claim 8 or 9, for a single daily administration.

**Patentansprüche**

**1.** Verbindungen der Formel (I):

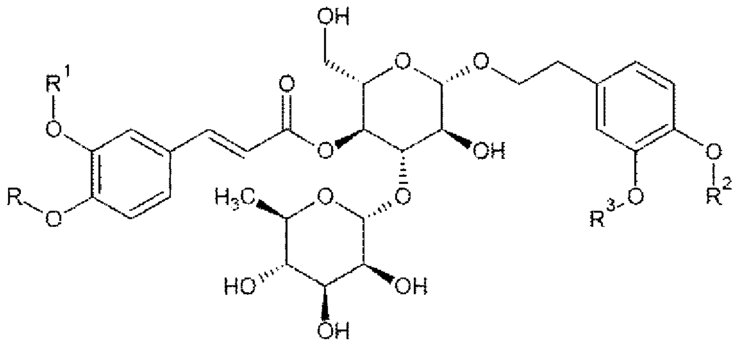

(I)

wobei R, $R^1$, $R^2$ und $R^3$ gleich oder verschieden sind und unabhängig voneinander ausgewählt sind aus Wasserstoff und einem Rückstand einer Carbonsäure, wobei die Estergruppen R-O-, $R^1$-O-, $R^2$-O- und $R^3$-O- jeweils bis zu einem pH-Wert im Bereich zwischen 4,5 und 7 hydrolysierbar sind, vorausgesetzt, dass R, $R^1$, $R^2$ und $R^3$ nicht gleichzeitig Wasserstoff sind; und
wobei mindestens eines von R, $R^1$, $R^2$ und $R^3$ ein Rückstand von Pivalinsäure ist.

**2.** Verbindungen der Formel (I) nach Anspruch 1, wobei die Estergruppen R-O-, $R^1$-O-, $R^2$-O- und $R^3$-O- bis zu einem pH-Wert im Bereich zwischen 5 und 6,5, oder einem pH-Wert zwischen 6 und 7 oder einem pH-Wert zwischen 6,3 und 6,7 hydrolysierbar sind.

**3.** Verbindungen der Formel (I) nach Anspruch 1 oder 2, wobei mindestens R und $R^1$ oder $R^2$ und $R^3$ gleichzeitig ein Rückstand von Pivalinsäure sind.

**4.** Verbindungen der Formel (I) nach Anspruch 3, wobei R und $R^1$ ein Rückstand von Pivalinsäure sind und $R^2$ und $R^3$ H sind, oder R und $R^1$ H sind und $R^2$ und $R^3$ ein Rückstand von Pivalinsäure sind.

**5.** Pharmazeutische Formulierung, enthaltend mindestens eine Verbindung der Formel (I) nach einem der Ansprüche 1 bis 4 und pharmazeutisch verträgliche Hilfsstoffe.

**6.** Verbindungen nach einem der Ansprüche 1 bis 4 oder Zusammensetzungen nach Anspruch 5 zur Verwendung als Thrombozyten-Antiaggregatoren.

**7.** Verbindungen der Formel (I) zur Verwendung nach Anspruch 6, wobei die Estergruppen R-O-, $R^1$-O-, $R^2$-O- und $R^3$-O- bis zu einem pH-Wert im Bereich zwischen 5 und 6,5, oder einem pH-Wert zwischen 6 und 7 oder einem pH-Wert zwischen 6,3 und 6,7 hydrolysierbar sind.

**8.** Verbindungen der Formel (I) zur Verwendung nach Anspruch 6 oder 7 bei der Behandlung von Diabetespatienten.

**9.** Verbindungen der Formel (I) zur Verwendung nach Anspruch 8 bei der Behandlung von Diabetespatienten, bei denen ein Risiko für Herz-Kreislauf-Erkrankungen besteht.

**10.** Verbindungen der Formel (I) zur Verwendung nach Anspruch 8 oder 9 für eine einzelne tägliche Verabreichung.

## Revendications

**1.** Composés de formule (I) :

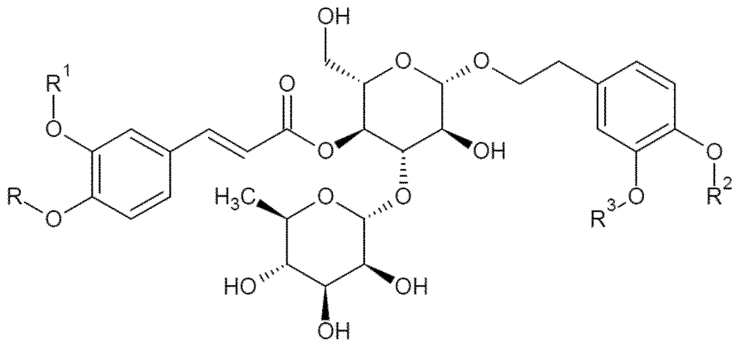

(I)

dans lesquels R, $R^1$, $R^2$ et $R^3$ sont identiques ou différents et sont indépendamment choisis parmi un hydrogène et un résidu d'un acide carboxylique, dans lesquels les groupements esters R-O-, $R^1$-O-, $R^2$-O- et $R^3$-O-, respectivement, sont hydrolysables à un pH compris entre 4,5 et 7, à la condition que R, $R^1$, $R^2$ et $R^3$ ne soit pas simultanément un hydrogène ; et
dans lesquels au moins l'un parmi R, $R^1$, $R^2$ et $R^3$ est un résidu d'acide pivalique.

**2.** Composés de formule (I) selon la revendication 1, dans lesquels les groupements esters R-O-, $R^1$-O-, $R^2$-O- et $R^3$-O-, respectivement, sont hydrolysables à un pH compris entre 5 et 6,5 ou à un pH compris entre 6 et 7 ou à un pH compris entre 6,3 et 6,7.

**3.** Composés de formule (I) selon la revendication 1 ou 2, dans lesquels au moins R et $R^1$ ou $R^2$ et $R^3$ sont simultanément des résidus d'acide pivalique.

**4.** Composés de formule (I) selon la revendication 3, dans lesquels R et $R^1$ sont des résidus d'acide pivalique et $R^2$ et $R^3$ sont H, ou R et $R^1$ sont H et $R^2$ et $R^3$ sont des résidus d'acide pivalique.

**5.** Formulation pharmaceutique contenant au moins un composé de formule (I) selon l'une quelconque des revendications 1 à 4 et des excipients pharmaceutiquement acceptables.

**6.** Composés selon l'une quelconque des revendications 1 à 4 ou composition selon la revendication 5 pour une utilisation comme antiagrégants plaquettaire.

**7.** Composés de formule (I) pour une utilisation telle que définie à la revendication 6, où les groupement esters R-O-, $R^1$-O-, $R^2$-O- et $R^3$-O-, respectivement, sont hydrolysables à un pH compris entre 5 et 6,5 ou à un pH compris entre 6 et 7 ou à un pH compris entre 6,3 et 6,7.

**8.** Composés de formule (I) pour une utilisation telle que définie à la revendication 6 ou 7, dans le traitement de patients diabétiques.

**9.** Composés de formule (I) pour une utilisation telle que définie à la revendication 8, dans le traitement de patients diabétiques ayant un risque de maladies cardiovasculaires.

**10.** Composés de formule (I) pour une utilisation telle que définie à la revendication 8 ou 9, en administration journalière unique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Remington's Pharmaceutical Sciences Handbook. Mack Pub. Co, 1985